# FASCICULE DE BREVET EUROPEEN

(11) **EP 1 933 808 B1**
(45) Date de publication et mention de la délivrance du brevet: **11.01.2017**
(21) Numéro de dépôt: 06831268.5
(22) Date de dépôt: 02.10.2006
(51) Int. Cl.: A61K 8/97, A61Q 19/08, A61Q 17/04

(54) **COMPOSITIONS COSMETIQUES NOTAMMENT A ACTIVITE ANTI-VIEILLISSEMENT COMPRENANT UN EXTRAIT DE LA PLANTE AFRAMOMUM ANGUSTIFOLIUM OU LONGOZA**
KOSMETISCHE ZUSAMMENSETZUNGEN, INSBESONDERE MIT ANTI-AGING-WIRKUNG, MIT EINEM EXTRAKT AUS DER LONGOSA-PFLANZE (AFRAMOMUM ANGUSTIFOLIUM)
COSMETIC COMPOSITIONS IN PARTICULAR WITH ANTI-AGING ACTIVITY COMPRISING AN EXTRACT OF AFRAMOMUM ANGUSTIFOLIUM OR LONGOZA PLANT

(30) Priorité: 04.10.2005 FR 0510147
(43) Date de publication de la demande: 25.06.2008
(73) Titulaire: LVMH RECHERCHE, 45800 St. Jean de Braye (FR)
(72) Inventeur: ANDRE, Patrice, F-45170 Neuville Aux Bois (FR); RENIMEL, Isabelle, F-45470 Trainore (FR); SAUVAN, Nancy, F-45000 ORLEANS (FR); RAZAFIMAMBIMBY, Hanitriniaina, 301 Fianarantsoa (MG)
(74) Mandataire: Portal, Gérard
(86) Numéro de dépôt international: PCT/FR2006/050982
(87) Numéro de publication internationale: WO 2007/042709

(56) Documents cités:
- E.M.GAYDOU: "Etude de la composition en acides gras des huiles extraites de graines provenant de quelques plantes de Madagascar" REVUE FRANCAISE DES CORPS GRAS , 30(1), 21-5 CODEN: RFCGAE; ISSN: 0035-3000, 1983, XP009068392 cité dans la demande
- BASER ET AL: "The essential oils of Aframomum corrorima (Braun) Jansen and A.angustifolium K.Schum. from Africa" MEDICINAL & AROMATIC PLANTS ABSTRACTS, NATIONAL INSTITUTE OF SCIENCE COMMUNICATION AND INFORMATION, IN, vol. 24, no. 4, août 2002 (2002-08), XP018007823 ISSN: 0250-4367
- JOHN B GITHIORI: "Evaluation of Anthelmintic Properties of Ethnovetinary Plant Preparations Used as Livestock Dewormers by pastoralists and Small Holder Farmers in Kenya" 2004, EPSILON , SWEDEN , XP002387646 Extrait de l'Internet: URL:http://web.archive.org/web/20041105081 852/http://diss-epsilon.slu.se/view/author /Githiori,_John_B..html> page 30 - page 32
- L.HARI, J.BUKURU, H.L.DE POOTER: "The Volatile Fraction of Aframomum sanguineum (K.Schum) K.Schum. from Burundi" JOURNAL OF ESSENTIAL OIL RESEARCH, vol. 6, 1994, pages 395-398, XP009068487
- T.J.COOMES: "Aframomum angustifolium seed from Zanzibar" COLONIAL PLANT AND ANIMAL PRODUCTS , 5(NO. 1), 68-77 CODEN: CPAPA4; ISSN: 0413-7442, 1955, XP009068490
- D.COUSINS: "Grands Singes Africains et Ethnomédicine" GORILLA JOURNAL, [Online] vol. 25, 2002, pages 9-11, XP002387644 Extrait de l'Internet: URL:http://www.berggorilla.org/english/gjo urnal/texte/25cousins.html> [extrait le 2006-06-26]
- DATABASE CA [Online] CHEMICAL ABSTRACTS SERVICE, COLUMBUS, OHIO, US; 1939, TRABAUD, L. ET AL: "Madagascan longoza oil. Scented species of Hedychium" XP002387849 extrait de STN Database accession no. 1939:21280 & PERFUMERY AND ESSENTIAL OIL RECORD, 29, 142-5 CODEN: PEORAA; ISSN: 0369-8998, 1938,
- P.DE LA GORCE, P.RASOANAIVO: "Essential Oils of Economic Value in Madagascar: Present State of Knowledge" HERBALGRAM, vol. 43, 1998, pages 31-39, XP002387645

## Description

La présente invention concerne essentiellement une composition cosmétique, notamment à activité anti-vieillissement, caractérisée en ce qu'elle comprend, à titre d'ingrédient actif, un extrait de graines de la plante *Aframomum angustifolium* ou *Longoza.*

L'invention concerne également l'utilisation d'un extrait de graines de la plante *Aframomum angustifolium* ou *Longoza,* comme agent cosmétique, notamment pour la fabrication d'une composition cosmétique, notamment ayant une activité anti-vieillissement.

Ci-après dans la description et les revendications, on utilisera de préférence par simplification le terme de *Longoza,* mais qui signifie évidemment indifféremment aussi la plante *Aframomum angustifolium.*

### ETAT DE LA TECHNIQUE

On connaît par l'article de E.M. Gaydou et al. paru dans la Revue française des Corps Gras, N°1 de janvier 1983, pages 21 à 25, une étude sur la composition en acide gras des huiles extraites de graines provenant de quelques plantes de Madagascar, dont la plante *Aframomum angustifolium* (voir tableau 1, suite 2, page 24). Il s'agit d'un extrait au moyen d'un solvant apolaire des graines de la plante, en particulier à l'aide de l'hexane (voir page 24, "Partie expérimentale", 1er paragraphe).

Cependant, aucune utilisation cosmétique, donc aucune composition cosmétique, n'est divulguée.

Le document BASER dans Phytochemistry, abrégé 2002-04-2340, divulgue des huiles essentielles distillées à l'eau à partir des graines *d'Aframomum corrorima* et Aframomum angustifolium.

Cependant, aucune utilisation cosmétique, donc aucune composition cosmétique, n'est divulguée.

La thèse de doctorat de l'Université suédoise de John B. GITHIORI (2004, EPSILON, SWEDEN) sur l'évaluation des propriétés antihelminthiques de préparation de plante ethnovétérinaire, cite en page 32 le *Longoza* et décrit à la page 31 la plante *Aframomum sanguineum,* qui est apparemment aussi un *Longoza* et pour laquelle on a effectué des extraits aqueux (page 31, dernier paragraphe).

A la page 32, il est précisé, pour cette plante, l'utilisation des graines pour faire un produit antihelminthique puissant pour les êtres humains. La plante est aussi connue pour réaliser un traitement pour les aigreurs d'estomac et la décoction des racines comme remède contre la dysenterie et les morsures de serpent. Les graines peuvent également être broyées et bouillies pendant 30 minutes avec 200 ml apparemment d'eau.

Dans ces conditions, ce document décrit un extrait aqueux et aucune utilisation cosmétique, donc aucune composition cosmétique, n'est divulguée.

Le document L. HARI, J. BUKURU, H.L. de Pooter dans J. Essent. Oil Res., 395-398 (Juillet/Août 1994) intitulé "The volatile fraction of Aframomum sanguineum*"* est aussi relatif à l'obtention d'huile essentielle *d'Aframomum sanguineum* par hydro-distillation ou par distillation à la vapeur, c'est-à-dire un extrait aqueux.

Cependant, une utilisation comme pommade cosmétique préparée par broyage des graines séchées et mélangée avec du beurre frais est, est divulguée dans l'introduction.

Le document Colonial plant and animal products, 5 (N°1), 68-77, CODEN, concerne l'article de COOMES et al. relatif à la plante *Aframomum angustifolium* et en particulier de ses graines de Zanzibar.

Il est dit à la page 69, premier paragraphe, que les graines ont été broyées dans un désintégrateur par choc et filtrées au travers d'une grille de mailles en acier 1/16 inch et soumises à une distillation à la vapeur sous agitation. Il s'agit donc d'une extraction aqueuse.

Le deuxième paragraphe parle d'ailleurs de la présence de globules d'huile détectés dans le distillat aqueux.

Aucune utilisation cosmétique, donc aucune composition cosmétique, n'est divulguée.

L' abrégé DATA BASE CA [Online] CHEMICAL ABSTRACTS SERVICE, COLUMBUS, OHIO, US ; 1939, TRABAUD, L. ET AL : «Madagascan longoza oil. Scented species of Hedychium» extrait de STN Database accession no. 1939:21280 est relatif à la Revue "Perfumery and Essential Oil Record", (1938), 29, 142-5, sur la plante *Longoza,* dont les fleurs sont extraites avec du pétrole léger (light petroleum) (voir ligne 3). L'huile obtenue est ensuite solubilisée dans 90% d'éthanol pour donner par distillation à la vapeur une huile jaune paille. Il s'agit au départ d'une extraction purement organique.

Aucune utilisation cosmétique, donc aucune composition cosmétique, n'est divulguée.

Enfin, La revue HerbalGram N°43, pages 31 à 59, sur les huiles essentielles de valeur économique de Madagascar, il est précisé pour Longoza en page 37, colonne de droite, l'obtention d'huiles essentielles à partir de rhizomes à raison de 0,17 à 0,7 %.

Il n'est pas clair dans cet article quelle a été la méthode d'extraction de l'huile essentielle de *Longoza.*

De même, aucune utilisation cosmétique, donc aucune composition cosmétique, n'est divulguée.

### BUTS DE L'INVENTION

L'invention a pour but de résoudre le problème technique consistant en la fourniture d'une solution permettant de trouver de nouveaux principes actifs comme agents cosmétiques ayant une bonne activité anti-vieillissement, ou une activité anti-vieillissement améliorée par rapport aux agents cosmétiques anti-vieillissement antérieurement connus, et/ou ayant une très bonne compatibilité dans le cadre d'une application topique dans un but cosmétique, notamment sur la peau, et/ou n'ayant pas ou sensiblement pas d'effets secondaires ou irritants.

L'invention a pour but de résoudre le problème technique énoncé ci-dessus à l'aide d'un principe actif obtenu à partir d'une source naturelle largement disponible ou aisément cultivable.

L'invention a encore pour but de résoudre le problème technique énoncé ci-dessus en fournissant aussi un procédé d'obtention du principe actif permettant de préparer des quantités suffisantes pour une utilisation à l'échelle industrielle et notamment à l'échelle cosmétique.

### RESUME DE L'INVENTION

L'invention apporte une solution aux problèmes techniques énoncés ci-dessus, d'une manière simple, peu coûteuse utilisable à l'échelle industrielle et notamment à l'échelle cosmétique.

Selon un premier aspect, la présente invention fournit une composition cosmétique, caractérisée en ce qu'elle comprend, à titre d'ingrédient actif, un extrait de graines de la plante *Aframomum angustifolium* ou *Longoza,* obtenu par un procédé d'extraction comprenant au moins une étape d'extraction des graines broyées avec un solvant alcoolique ou hydroalcoolique, dans un excipient cosmétiquement acceptable.

Selon un second aspect, l'invention concerne également un agent cosmétique sous forme d'un extrait de graines de la plante *Aframomum angustifolium* ou *Longoza,*tel que défini selon le premier aspect.

Selon un mode de réalisation avantageux de l'invention, l'extrait de graines de *Aframomum angustifolium* ou *Longoza* est utilisé pour une activité anti-vieillissement.

Selon une variante de réalisation avantageuse de l'invention, on utilise l'extrait de graines de la plante *Longoza* pour une activité anti-rides, notamment pour diminuer, éliminer, ou ralentir l'apparition de rides, une action préservant ou restaurant la structure de la peau, notamment par une action de stimulation de la synthèse de collagène, en particulier de collagène VII présent au niveau de la jonction dermo-épidermique ou JDE, et/ou par une action sur les fibres oxytalanes et/ou les fibres élaunines. Ainsi, l'invention permet d'avoir un effet anti-rides ; un effet de protection, de réparation ou de restauration de l'élasticité et/ou de la fermeté de la peau. L'invention permet aussi de protéger la peau contre un vieillissement résultant de l'action des rayonnements actiniques, notamment dû aux rayons ultraviolets.

De préférence, le broyage est réalisé jusqu'à obtenir une poudre fine. Les grains de cette poudre présentent avantageusement un diamètre compris entre 0,01 et 1 mm environ.

Selon un mode de réalisation avantageux du procédé selon l'invention, on utilise un solvant alcoolique ou hydroalcoolique dans lequel l'alcool est un mono-alcool - ou un polyol comprenant de 1 à 4 atomes de carbone. Avantageusement, cet alcool est choisi parmi le méthanol, l'éthanol, le n-propnol, l'isopropanol, l'éthylèneglycol, le propylène glycol et le butylène-glycol, de préférence l'éthanol. On peut procéder à l'opération d'extraction soit à la température ambiante, soit à une température pouvant aller jusqu'à la température d'ébullition du solvant à la pression atmosphérique, ou même sous une pression plus élevée de sorte toutefois que la température d'ébullition ne dépasse pas environ 120°C. La proportion relative entre l'alcool et l'eau est choisie de préférence dans une fourchette variant de 30/70 à 100/0 en volume.

On peut procéder à plusieurs extractions successives jusqu'à épuisement de la matière à extraire par le solvant considéré.

La durée d'extraction varie en fonction du solvant considéré, la température et éventuellement de la pression utilisée, pour aboutir à l'épuisement total d'extraction de la matière. En pratique, cette durée sera limitée à moins d'une heure pour une exploitation industrielle rentable. Elle sera généralement de l'ordre de 30 minutes.

Selon une variante de réalisation particulière de ce procédé d'extraction, l'extrait alcoolique ou hydroalcoolique obtenu est avantageusement délipidé par élimination des lipides par au moins une étape d'extraction avec un solvant organique apolaire, par exemple l'hexane, ou le n-heptane, en obtenant ainsi un extrait alcoolique ou hydroalcoolique délipidé.

Selon un mode particulier de réalisation de ce procédé d'extraction, l'extrait alcoolique ou hydroalcoolique délipidé peut constituer en lui-même l'ingrédient ou l'agent actif selon l'invention.

Selon une autre variante de réalisation particulière du procédé d'extraction selon l'invention, l'extrait alcoolique ou hydroalcoolique délipidé peut être soumis à une étape de décoloration par filtration sur un agent décolorant approprié, tel que du charbon puis, après élimination du charbon, lavage avec une solution d'un solvant alcoolique ou hydroalcoolique qui peut être le même que celui qui a servi à l'extraction de départ, ou différent.

On peut, selon une autre variante particulière, évaporer sensiblement complètement le solvant d'extraction en obtenant un extrait sec que l'on peut soit utiliser tel quel comme l'ingrédient actif précité, soit dissoudre à nouveau dans un solvant cosmétiquement acceptable, en particulier un alcool ou un mélange hydro-alcoolique, afin d'obtenir une autre variante de l'ingrédient actif précité.

Selon une autre variante avantageuse de l'invention, l'alcool utilisé pour l'extraction est l'éthanol.

Dans le cadre de l'invention, l'extrait obtenu est facile à utiliser comme agent cosmétique et à mélanger avec les autres ingrédients de composition cosmétique à préparer, que ce soit pour incorporer dans une phase aqueuse ou une phase grasse.

Selon un troisième aspect, la présente invention concerne aussi un extrait de graines de la plante *Aframomum angustifolium* ou *Longoza* tel que précédemment défini utilisé dans un procédé de soin cosmétique par application topique sur la peau.

Selon un mode de réalisation avantageux de l'invention, ce procédé de soin cosmétique est mis en oeuvre pour appliquer l'extrait de graines de la plante *Aframomum angustifolium* ou *Longoza* précité sur les zones de la peau, ayant besoin d'un soin anti-vieillissement, en particulier pour lutter contre les effets du vieillissement résultant de l'action des rayonnements actiniques, notamment des rayons ultraviolets.

Plus précisément, l'activité anti-vieillissement vise particulièrement une activité anti-rides, pour diminuer, éliminer, ou ralentir l'apparition de rides.

L'invention a aussi une action préservant ou restaurant la structure de la peau, notamment par une action de stimulation de la synthèse de collagène, en particulier de collagène VII, présent au niveau de la jonction dermo-épidermique, ou JDE et/ou par une action réparatrice sur les fibres oxytalanes et/ou les fibres élaunines, un effet de protection, de réparation ou de restauration de l'élasticité et/ou de la fermeté de la peau.

Dans le cadre de l'un quelconque des aspects de l'invention, ledit extrait est présent à une concentration, exprimée en extrait sec, comprise entre 0,001 % et 5 %, de préférence entre 0,01 % et 1%, en poids par rapport au poids total de la composition.

D'autres buts, caractéristiques et avantages de l'invention apparaîtront clairement à l'homme de l'art à partir de la description suivante faite en référence aux dessins annexés, dans lesquels :

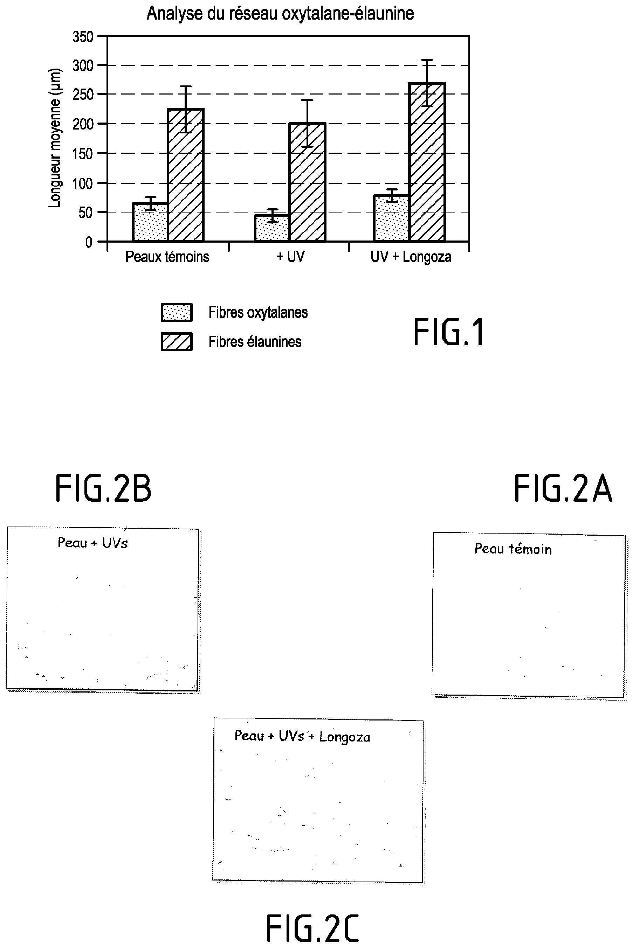

- la figure 1 représente les résultats d'essais obtenus avec l'extrait de graines de la plante *Aframomum angustifolium* ou *Longoza* sur peau humaine, maintenue en survie, vieillie artificiellement par rayonnement ultraviolet (résultats rapportés sous la dénomination «UV + *Longoza*»), par quantification histologique des fibres élastiques par analyse d'image informatisée par analyse du réseau oxytalane-élaunine, en comparaison avec la même peau humaine formant une peau témoin (résultats rapportés sous la dénomination «Peaux témoins», ou la même peau humaine, vieillie artificiellement par rayonnement UV (dite «+ UV», rapportées en abscisses ; avec en ordonnées l'indication de la longueur moyenne des fibres élastiques en micromètres sur une échelle allant de 0 à 350 ;
- la figure 2A montre une photo prise au microscope électronique avec un grossissement de 400 fois de la peau témoin ; la figure 2B montre une photo prise dans des conditions identiques sur la peau ayant subi un vieillissement artificiel au rayonnement UV ; et la figure 2C montre les résultats obtenus en combinant un vieillissement par les UV en présence d'un extrait alcoolique ou hydroalcoolique de graines de *Longoza* selon l'invention préparée selon l'exemple 1 ci-après, montrant dans le cadre de l'invention une restauration du réseau oxytalane-élaunine et des fibres élastiques matures ;

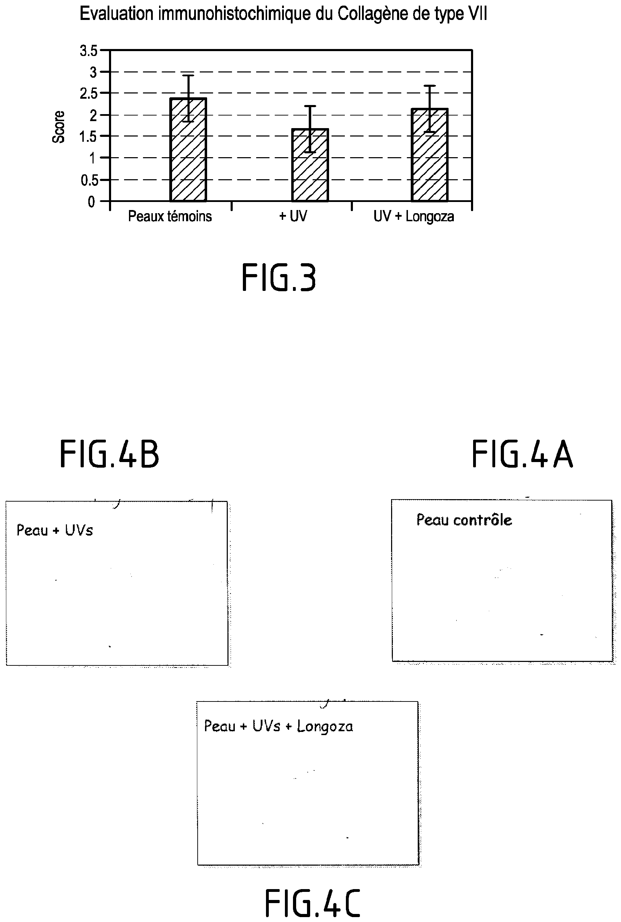

- la figure 3 représente les résultats de mesure d'évaluation immunohistochimique du collagène de type VII se trouvant au niveau de la jonction dermo-épidermique avec, en abscisses, respectivement les résultats obtenus avec les "Peaux témoins" ; avec ces peaux soumises aux UV uniquement (dites "+ UV") ; et avec ces peaux ayant subi le traitement aux ultraviolets en présence du même extrait de *Longoza* selon l'invention ("UV + *Longoza"*); et en ordonnées, le score obtenu selon une échelle variant de 0 à 3,5, mettant en évidence un effet réparateur significatif au niveau du collagène VII grâce à l'extrait de l'invention de *Longoza* par rapport à la peau vieillie expérimentalement lors de la soumission du rayonnement aux ultraviolets ;
- la figure 4A est une photo montrant une coupe de la peau au niveau de la jonction dermo-épidermique montrant l'intensité du marquage du collagène VII au niveau de la Jonction Dermo-Epidermique ou JDE («Peau contrôle»); la figure 4B représente dans les mêmes conditions, la peau ayant été soumise aux rayonnements ultraviolets («Peau+UVs») ; la figure 4C représente la même peau ayant subi le rayonnement ultraviolet en présence de l'extrait de graines de *Longoza* selon l'invention («Peau + UVs + Longoza»), montrant qu'avec l'extrait de *Longoza* selon l'invention, on observe une intensité de marquage proche de celle du témoin sans UV.

Dans les exemples ci-après, tous les pourcentages sont donnés en poids, la température est la température ambiante, soit 25°C, ou est donnée en degré Celsius, la pression est la pression atmosphérique, sauf indication contraire.

### Exemple 1

### Exemple de préparation d'un extrait de graines de la plante Aframomum angustifolium ou Longoza, alcoolique ou hydroalcoolique, délipidé, selon l'invention

A partir de 100 kg de graines de la plante *Longoza* de différents lots du commerce, disponibles auprès de la Société SOTRAMEX, France, on réalise la procédure d'extraction suivante :
a) tout d'abord, on effectue un broyage des graines séchées du commerce jusqu'à par exemple une dimension moyenne de poudre comprise entre 0.01 mm et 1 mm.
b) ensuite, on procède à trois extractions successives des graines broyées avec à chaque fois 500 Litres (L) d'un mélange hydroalcoolique (par exemple un mélange éthanol/eau dans un rapport volumique 70/30), avantageusement sous un léger chauffage, par exemple à 50°C, sous agitation, pendant une durée d'environ 30 minutes. L'alcool préféré est l'éthanol pour sa bio-compatilité bien connue. Il est bien entendu que l'extraction pourrait aussi bien être réalisée avec de l'alcool pur.
   On procède à une filtration du gâteau d'extraction après chaque extraction et également à un lavage. La filtration a par exemple lieu sur filtre ayant un diamètre de pores de 0,70 µm, le lavage ayant lieu avec 30 L de mélange hydroalcoolique à 70/30 volume/volume.
c) On procède ensuite à un regroupement des filtrats en obtenant ainsi un volume total de filtrat d'environ 1 500 L, la solution obtenue étant trouble.
d) On procède ensuite avantageusement à une étape d'élimination des lipides ou délipidation par extraction liquide/liquide avec un solvant organique apolaire, par exemple le n-heptane.
   Avantageusement, on procède à trois extractions avec à chaque fois 500 L environ de n-heptane sous agitation vive.
   On procède à une décantation pour obtenir la séparation de la phase heptanique de la phase hydroalcoolique, la phase heptanique étant rejetée.
   La phase hydroalcoolique délipidée ainsi obtenue est quasiment limpide et présente un volume d'environ 1500 L.
   Cette phase hydroalcoolique délipidée constitue en elle-même selon le mode de réalisation actuellement préféré l'extrait de graine de *Longoza* ou de *Aframomum angustifolium* selon l'invention utilisable comme agent cosmétique ou à titre d'ingrédient actif pour la fabrication d'une composition cosmétique comme montré dans les exemples suivants (extrait 1d). Cette solution hydroalcoolique contient 0,47 % en poids d'extrait sec.
   Il est entendu qu'en modifiant les paramètres d'extraction, tels que la quantité de solvant d'extraction utilisée est la proportion d'alcool dans le mélange hydroalcoolique, il est possible d'obtenir des teneurs différentes en extrait sec, par exemple plus élevées, notamment de 1 % en poids environ la concentration en poids en extrait sec peut varier dans certaines limites.
e) Selon une variante de réalisation, on peut procéder à une étape d'élimination des traces de solvant organique apolaire utilisée, tel que le n-heptane, par exemple par distillation en chauffant la phase hydroalcoolique à une température de 50°C maximum, éventuellement sous pression réduite.
f) On peut procéder ensuite à une dilution de la solution alcoolique ou hydroalcoolique délipidée ne comportant plus de solvant organique apolaire, jusqu'à retrouver le volume de 1 500 L en ajoutant de la solution de solvant alcoolique ou hydroalcoolique.
g) On peut encore procéder à une décoloration de la solution délipidée par traitement sur du charbon comme cela est bien connu à l'homme de l'art, pendant 1h à la température ambiante, sous agitation.
   On peut ensuite procéder à une filtration du charbon sur un filtre ayant un diamètre de pores de 0,45 µm puis à un lavage du charbon filtré avec 3 L de solution alcoolique ou hydroalcoolique.
   La solution alcoolique ou hydroalcoolique délipidée et décolorée est limpide et présente un volume d'environ 1500 L.
h) On peut procéder ensuite à une concentration de ladite solution par l'évaporation principalement de l'alcool afin d'obtenir une phase essentiellement aqueuse, notamment par chauffage à une température modérée de 50°C maximum, éventuellement sous pression réduite. Cette solution essentiellement aqueuse pourra également être utilisée en l'état comme ingrédient actif selon l'invention.
i) Selon encore une autre variante de réalisation, on peut procéder à une lyophilisation de ladite solution essentiellement aqueuse, pour obtenir un extrait sec pouvant également être utilisé tel quel comme ingrédient actif selon l'invention.
j) pour l'incorporation de cet extrait sec dans une composition cosmétique selon l'invention, il est aussi possible d'en préparer une solution extemporanée au moment de fabriquer ladite composition, dans un solvant cosmétiquement acceptable, notamment dans de l'eau, de l'alcool, du butylèneglycol 1-3 ou dans un mélange de ces solvants. Ce mode de réalisation permet ainsi de préparer des solutions d'extrait sec de la plante *Aframomum angustifolium* ou *Longoza* à la concentration voulue.
k) on peut enfin procéder à une opération de conditionnement des différentes solutions de l'extrait décrites ci-dessus, dans l'attente de leur utilisation pour la fabrication des compositions cosmétiques recherchées. Dans ce cadre, il est préféré de réaliser préalablement une filtration stérilisante sur filtre ayant un diamètre de pores de 0,22 µm avant le conditionnement dans des flacons de volumes appropriés, puis le stockage dans une chambre froide, par exemple à 4° C.

### Exemple 2 selon l'invention

### Détermination de l'activité anti-vieillissement de l'extrait de Longoza (Aframomum angustifolium) sur un mode expérimental de peau humaine maintenue en survie et vieillie artificiellement par rayonnement UV

Pour déterminer l'activité anti-vieillissement de l'extrait de *Longoza* selon l'invention, il a été utilisé un modèle expérimental de peau maintenue en survie et vieillie artificiellement par rayonnement ultraviolet («UV») afin d'évaluer l'effet anti-âge.

Cet essai a été évalué par analyse de la restauration de la qualité de la jonction dermo-épidermique par évaluation immunohistochimique du collagène VII, et des fibres élastiques du derme superficiel par étude du réseau oxytalane-élaunine, et du derme moyen-profond par quantification morphométrique par analyse d'image.

Pour ce faire, des fragments de peau ont été prélevés chez des femmes après chirurgie plastique à partir de huit donneurs différents. Ces fragments sont déposés dans des inserts eux-mêmes disposés en suspension au-dessus des puits de culture.

Du milieu de culture MEM bien connu de l'homme de l'art, complété avec un antibiotique tel que la gentamicyne, un extrait pituitaire bovin, du sérum de veau foetal et de l'hydrocortisone, est ajouté dans le fond des puits. Un passage du milieu de culture s'effectue par diffusion depuis la base des puits au travers d'une membrane poreuse d'une épaisseur de 12 µm. Ce milieu de culture a été renouvelé tous les trois jours.

Un modèle expérimental de vieillissement est réalisé en prévoyant deux séances d'irradiation aux ultraviolets, comprenant une composante en UVA (8 J/cm²) et en UVB (2 J/cm²), respectivement après un jour ("J1") et au troisième jour ("J3"), afin de provoquer des altérations cutanées proches de celles observées au cours du vieillissement.

On prépare une solution à 5 % dans le milieu de culture de la solution d'extrait de Longoza obtenu en fin d'étape d) à l'exemple 1 ci-dessus (extrait 1d). Du premier jour ("J0") au dixième jour ("J10"), on applique cette solution à 5 % à la surface des fragments de peau, trois fois par semaine.

Dans le cadre de cet essai, une comparaison est donc effectuée entre :
a) une peau témoin mise simplement en survie ;
b) une peau maintenue en survie, vieillie expérimentalement par les UVA et B (peau contrôle de référence) ;
c) une peau maintenue en survie, vieillie artificiellement aux ultraviolets A/B, avec application de l'extrait de *Longoza* comme indiqué ci-dessus.

Certaines cultures de peau sont stoppées au quatrième jour ("J4") pour l'analyse immunohistochimique du collagène VI et d'autres le dernier jour ("J10"), pour l'analyse morphométrique des fibres élastiques. Les résultats obtenus sont les suivants :

### 1) Quantification histologique des fibres élastiques

Les fibres élastiques sont révélés par une coloration à la (+) catéchine et quantifiés morphométriquement par analyse d'image informatisée fournie par une caméra XC-75CE LEITZ, assistée par ordinateur équipé d'un logiciel fourni par le fabriquant de la caméra.

La longueur moyenne (µm) des fibres oxytalanes et élaunines du derme superficiel est évaluée, ainsi que leur surface moyenne (µm²).

Dans le cadre des résultats réalisés par les inventeurs, il a pu être mis en évidence, avec le modèle de vieillissement de la peau utilisée, une diminution statistiquement significative de la longueur moyenne des fibres oxytalanes (42,7 µm) et de la surface moyenne des fibres oxytalanes et élaunines du derme (2339 µm²) par rapport à la peau témoin (longueur 64 µm et surface 2808,9 µm²), constituant une valeur statistiquement significative avec un p < 0,05.

Les résultats sont répertoriés à la figure 1 annexée et les photos prises au microscope électronique LEITZ équipé de la caméra XC-75CE LEITZ, font l'objet des figures 2A (peau témoin), 2B (peau + UV), 2C (Peau + UV + Longoza).

On observera que l'effet réparateur au niveau des fibres oxytalanes et élaunines est significatif avec l'extrait selon l'invention de *Longoza* tel qu'obtenu à l'exemple 1, sous forme d'extrait alcoolique ou hydroalcoolique délipidé.

En effet, la longueur moyenne des fibres oxytalanes des peaux traitées par l'extrait de *Longoza* selon l'invention est de 75 µm (p < 0,05). La longueur moyenne des fibres élaunines est augmentée à 267,4 µm par rapport à la peau vieillie expérimentalement dont la longueur est réduite à 200 µm. De même, la surface moyenne des fibres élaunines est significativement augmentée à 3 397,2 µm² avec un écart p < 0,05.

Ainsi, l'effet réparateur au niveau des fibres élastiques matures du derme moyen et profond est lui aussi significatif par rapport au témoin + ultraviolet (+7,7 % pour le derme moyen et +8,4 % pour le derme profond).

Tous ces résultats sont clairement visibles à la figure 1 annexée.

### 2) Analyse immunohistochimique du collagène de type VII

A partir des fragments de peau prélevés chez des femmes après chirurgie plastique comme indiqué précédemment, congelés, il est possible de détecter par immunohistochimie le collagène de type VII situé au niveau de la membrane basale.

L'immunodétection est réalisée à l'aide d'une technique d'immunoperoxydase indirecte en trois couches à l'aide d'un kit disponible dans le commerce dénommé ABC Peroxydase, disponible chez Vector Laboratories, USA, et révélé avec un agent révélateur tel que la DAB (diaminobenzidine).

L'évaluation est effectuée à l'aide d'une notation semi-quantitative allant de 0 à 3, précisant l'intensité du marquage et sa topographie, c'est-à-dire l'extension plus ou moins importante au niveau de la Jonction Dermo-Epidermique.

Le modèle expérimental aux ultraviolets précité de vieillissement de la peau a permis d'altérer le collagène VII au niveau de la Jonction Dermo-Epidermique (JDE).

Il a été obtenu en effet une notation de 1,67 par rapport à 2,4 pour la peau témoin.

Cette différence est statistiquement significative avec un écart p < 0,05.

Un effet réparateur significatif est observé au niveau du collagène VII lorsqu'on utilise l'extrait de *Longoza* selon l'invention, par rapport à la peau vieillie expérimentalement aux ultraviolets dans les conditions précitées, avec un écart p < 0,05.

Les résultats de cette évaluation immunohistochimique du collagène de type VII, qui ont été obtenus, sont répertoriés à la figure 3 annexée.

D'autre part, à la figure 3A, il est présenté une photo prise au microscope électronique d'une coupe de peau contrôle, la figure 3B une même photo pour une peau vieillie artificiellement aux ultraviolets, et, la figure 3C une photo prise dans les mêmes conditions pour la peau vieillie artificiellement par les ultraviolets et avec traitement de la peau par l'extrait de *Longoza* selon l'invention.

On observe, en présence d'UV, une diminution d'intensité du marquage du collagène VII au niveau de la JDE.

Par contre, lorsqu'il y a traitement de la peau par l'extrait de *Longoza* selon l'invention, comme indiqué précédemment, on observe une intensité de marquage proche de celle du témoin sans UV, montrant l'efficacité de protection de l'extrait de *Longoza* selon l'invention.

En conclusion de ce qui précède, grâce au modèle de vieillissement expérimental utilisé, sur peau humaine maintenue en survie, il a pu être mis en évidence l'effet significativement réparateur de l'extrait de *Longoza* selon l'invention sur les fibres oxytalanes et élaunines du derme superficiel, sur le réseau élastique du derme moyen et profond et sur le collagène VII de la jonction dermo-épidermique.

Ainsi, l'invention peut être utilisée comme agent cosmétique pour la préparation de composition cosmétique à usage topique en vue de préserver les protéines de structure comme le collagène, l'élastine, ainsi que pour réaliser un renforcement de la jonction dermo-épidermique.

Divers exemples de formulation de composition cosmétique sont donnés ci-après à titre illustratif et qui ne sauraient donc en aucune façon limiter la portée de l'invention.

Dans tous les exemples de la description, tous les pourcentages sont donnés en poids, sauf indication contraire.

### Exemple 3 selon l'invention

### Gel cosmétique pour améliorer la fermeté du visage

- extrait alcoolique ou hydroalcoolique délipidé de *Longoza* obtenu à l'exemple 1, d) 2 %
- glycol 3 %
- polymère d'AMPS disponible dans le commerce, (dénomination commerciale Sepigel 305®) 3 %
- huile de ricin hydrogénée (Cremophor CO-60®) 2 %
- polyéthylène glycol 1,5 %
- conservateur 0,5 %
- concentré de parfum 0,3 %
- filtre UV (benzophénone-4) 1 %
- eau, qsp 100 %

Ce gel peut être appliqué une fois par jour pendant plusieurs semaines sur le visage afin d'aboutir à l'amélioration de la fermeté de la peau du visage, et notamment sur les zones comportant des rides, en observant ainsi à l'issue de ce traitement, une restauration de la souplesse avec un effet manifeste de rajeunissement de la peau et d'atténuation ou de disparition de ces rides.

### Exemple 4 selon l'invention

### Crème de jour anti-rides sous forme d'une émulsion

- extrait alcoolique ou hydroalcoolique délipidé de *Longoza* obtenu à l'exemple 1, d) 2 %
- steareth-21 (Brij 721) 2,5 %
- glycéryl stéarate (Tegrin) 1,1 %
- alcool stéarylique 5 %
- tricaprat / caprilate glycérol 12,5 %
- butylène glycol 3 %
- glycérine 2 %
- conservateur 0,5 %
- concentré de parfum 0,5 %
- filtre UV (méthoxycinnamate d'octyle) 7,5 %
- eau, qsp 100 %

### Exemple 5 selon l'invention

### Lotion tonique anti-rides

- extrait alcoolique ou hydroalcoolique délipidé de *Longoza* obtenu à l'exemple 1, d) 2 %
- butylène glycol 3 %
- EDTA 0,1 %
- solubilisant 1 %
- concentré de parfum 0,3 %
- éthanol 5 %
- filtre UV (benzophénone -4) 0,13 %
- eau, qsp 100 %

### Exemple 6 selon l'invention

### Poudre de maquillage protectrice des ultraviolets pour le visage

- extrait alcoolique ou hydroalcoolique délipidé de *Longoza* obtenu à l'exemple 1, d) 0,25 %
- talc 17 %
- mica 20 %
- sericite 20 %
- pigments 8 %
- poudre organique (nylon) 20 %
- silice 8,75 %
- huile minérale ou silicone 3 %
- filtre UV (méthoxycinnamate d'octyle) 3 %

### Exemple 7 selon l'invention

### Fond de teint traitant anti-rides

- extrait alcoolique ou hydroalcoolique délipidé de *Longoza* obtenu à l'exemple 1, d) 2 %
- polyglycéryl-4 isostéarate et cétyl diméthicone copolyol et hexyl laurate 5,1 %
- cyclopentasiloxane et cyclohexasiloxane 5,0 %
- cetyl diméthicone 1,0 %
- caprylic/capric triglycérides 2,2 %
- octyl stéarate 1,4 %
- huile minérale 3,5 %
- huile de ricin hydrogénée 1,2 %
- cire d'abeille 0,8 %
- polyméthacrylate de méthyle 1,1 %
- oxydes de fer 0,45 %
- dioxydes de titane 5,2 %
- NaCl 0,6 %
- concentré de parfum 0,1 %
- filtre UV (cinnamate d'octyle) 3 %
- eau, qsp 100 %

## Revendications

1. Composition cosmétique, **caractérisée en ce qu'**elle comprend, à titre d'ingrédient actif, un extrait de graines de la plante *Aframomum angustifolium obtenu par un procédé d'extraction comprenant au moins une étape d'extraction des graines broyées avec un solvant alcoolique ou hydroalcoolique,* dans un excipient cosmétiquement acceptable.

2. Composition selon la revendication 1 , **caractérisée en ce que** l'extrait alcoolique ou hydroalcoolique obtenu est délipidé par élimination des lipides par au moins une étape d'extraction avec un solvant organique apolaire, par exemple l'hexane, ou le n-heptane, en obtenant ainsi un extrait alcoolique ou hydroalcoolique délipidé.

3. Composition selon l'une des revendications 1 ou 2, **caractérisée en ce que** l'extrait alcoolique ou hydroalcoolique délipidé constitue en lui-même l'ingrédient actif.

4. Composition selon l'une des revendications 2 ou 3, **caractérisée en ce que** l'extrait alcoolique ou hydroalcoolique délipidé est soumis à une étape de décoloration par filtration sur un agent décolorant approprié, tel que du charbon puis, après élimination du charbon, lavage avec une solution d'un solvant alcoolique ou hydroalcoolique qui peut être le même que celui qui a servi à l'extraction de départ, ou différent.

5. Composition selon l'une des revendications 1 à 4, **caractérisée en ce qu'**on évapore sensiblement complètement le solvant d'extraction en obtenant un extrait sec que l'on peut soit utiliser tel quel, comme l'ingrédient actif précité, soit dissoudre à nouveau dans un solvant cosmétiquement acceptable, en particulier un alcool ou un mélange hydroalcoolique, afin d'obtenir une autre variante de l'ingrédient actif précité.

6. Composition selon l'une des revendications 1 à 5, **caractérisée en ce que** l'alcool précité ou l'alcool utilisé pour le mélange hydroalcoolique précité est un mono-alcool ou un polyol comprenant de 1 à 4 atomes de carbone.

7. Composition selon la revendication 6, **caractérisée en ce que** ledit alcool est choisi parmi le méthanol, l'éthanol, le n-propanol, l'isopropanol, l'éthylèneglycol, le polypropylène glycol et le butylène-glycol.

8. Composition selon la revendication 6 ou 7, **caractérisée en ce que** ledit alcool est l'éthanol.

9. Composition selon l'une des revendications 1 à 8, **caractérisée en ce que** l'extrait précité de graines de *Aframomum angustifolium* est présent à une concentration, exprimée en extrait sec, comprise entre 0,001 % et 5 %,, en poids par rapport au poids total de la composition.

10. Composition selon l'une des revendications 1 à 9, **caractérisée en ce que** l'extrait précité de graines de *Aframomum angustifolium* est présent à une concentration, exprimée en extrait sec, comprise entre 0,01% et 1%, en poids par rapport au poids total de la composition,

11. Agent cosmétique sous forme d'un extrait de graines de la plante *Aframomum angustifolium,* ledit extrait étant tel que défini à l'une quelconque des revendications 1 à 10.

12. Agent cosmétique selon la revendication 11, **caractérisée en ce que** l'extrait de *Aframomum angustifolium* est utilisé pour une activité anti-vieillissement.

13. Extrait de graines de la plante *Aframomum angustifolium* tel que défini à l'une quelconque des revendications 1 à 10 utilisé dans un procédé de soin cosmétique par application topique sur la peau.

14. Extrait selon la revendication 13, **caractérisé en ce qu'**on réalise l'application de l'extrait de graines de la plante *Aframomum angustifolium* précité sur les zones de la peau ayant besoin d'un soin anti-vieillissement, en particulier pour lutter contre les effets du vieillissement résultant de l'action des rayonnements actiniques, notamment des rayons ultraviolets.

15. Extrait selon la revendication 13, **caractérisé en ce que** l'activité anti-vieillissement vise particulièrement une activité anti-rides pour diminuer, éliminer, ou ralentir l'apparition de rides, une action préservant ou restaurant la structure de la peau, notamment par une action de stimulation de la synthèse de collagène, en particulier de collagène VII, en particulier présent au niveau de la jonction dermo-épidermique, ou JDE et/ou par une action réparatrice sur les fibres oxytalanes et/ou les fibres élaunines, un effet de protection, de réparation ou de restauration de l'élasticité et/ou de la fermeté de la peau.

## Patentansprüche

1. Kosmetische Zusammensetzung, **dadurch gekennzeichnet, dass** sie als wirksamen Bestandteil einen Extrakt aus Samen der Pflanze *Aframomum angustifolium,* welcher durch ein Extraktionsverfahren, das wenigstens einen Schritt des Extrahierens der gemahlenen Samen mit einem alkoholischen oder wässrig-alkoholischen Lösungsmittel umfasst, erhalten wird, in einem kosmetisch akzeptablen Hilfsstoff umfasst.

2. Zusammensetzung nach Anspruch 1, **dadurch gekennzeichnet, dass** der erhaltene alkoholische oder wässrig-alkoholische Extrakt durch Entfernen der Fette mittels wenigstens eines Extraktionsschrittes mit einem organischen, apolaren Lösungsmittel, zum Beispiel Hexan, oder n-Heptan, entfettet wird, wodurch ein entfetteter alkoholischer oder wässrig-alkoholischer Extrakt erhalten wird.

3. Zusammensetzung nach einem der Ansprüche 1 oder 2, **dadurch gekennzeichnet, dass** der entfettete alkoholische oder wässrig-alkoholische Extrakt an sich den wirksamen Bestandteil bildet.

4. Zusammensetzung nach einem der Ansprüche 2 oder 3, **dadurch gekennzeichnet, dass** der entfettete alkoholische oder wässrig-alkoholische Extrakt einem Entfärbungsschritt durch Filtration über einem geeigneten Entfärbungsmittel, wie Kohle, anschließend, nach Entfernen der Kohle, Waschen mit einer Lösung eines alkoholischen oder wässrig-alkoholischen Lösungsmittels, welches das gleiche wie das sein kann, das der Ausgangsextraktion diente, oder verschieden sein kann, unterzogen wird.

5. Zusammensetzung nach einem der Ansprüche 1 bis 4, **dadurch gekennzeichnet, dass** das Extraktionslösungsmittel unter Erhalten eines Trockenextraktes im Wesentlichen vollständig verdampft wird, welcher entweder unverändert als der vorgenannte wirksame Bestandteil verwendet werden kann oder erneut in einem kosmetisch akzeptablen Lösungsmittel, insbesondere einem Alkohol oder einem wässrig-alkoholischen Gemisch gelöst werden kann, um eine andere Variante des vorgenannten wirksamen Bestandteils zu erhalten.

6. Zusammensetzung nach einem der Ansprüche 1 bis 5, **dadurch gekennzeichnet, dass** der vorgenannte Alkohol oder der Alkohol, der für das vorgenannte wässrig-alkoholische Gemisch verwendet wird, ein einwertiger Alkohol oder ein Polyol mit 1 bis 4 Kohlenstoffatomen ist.

7. Zusammensetzung nach Anspruch 6, **dadurch gekennzeichnet, dass** der Alkohol aus Methanol, Ethanol, n-Propanol, Isopropanol, Ethylenglykol, Polypropylenglykol und Butylenglykol ausgewählt ist.

8. Zusammensetzung nach Anspruch 6 oder 7, **dadurch gekennzeichnet, dass** der Alkohol Ethanol ist.

9. Zusammensetzung nach einem der Ansprüche 1 bis 8, **dadurch gekennzeichnet, dass** der vorgenannte Extrakt aus Samen von *Aframomum angustifolium* in einer Konzentration, ausgedrückt in Trockenextrakt, zwischen 0,001 und 5 Gew.-% bezogen auf das Gesamtgewicht der Zusammensetzung vorliegt.

10. Zusammensetzung nach einem der Ansprüche 1 bis 9, **dadurch gekennzeichnet, dass** der vorgenannte Extrakt aus Samen von *Aframomum angustifolium* in einer Konzentration, ausgedrückt in Trockenextrakt, zwischen 0,01 und 1 Gew.-% bezogen auf das Gesamtgewicht der Zusammensetzung vorliegt

11. Kosmetischer Wirkstoff in Form eines Extraktes aus Samen der Pflanze *Aframomum angustifolium,* wobei der Extrakt so ist wie in einem der Ansprüche 1 bis 10 definiert.

12. Kosmetischer Wirkstoff nach Anspruch 11, **dadurch gekennzeichnet, dass** der Extrakt von *Aframomum angustifolium* wegen einer Anti-Aging-Aktivität verwendet wird.

13. Extrakt aus Samen der Pflanze *Aframomum angustifolium,* wie in einem der Ansprüche 1 bis 10 definiert, der bei einem Verfahren zur kosmetischen Pflege durch topische Anwendung auf der Haut verwendet wird.

14. Extrakt nach Anspruch 13, **dadurch gekennzeichnet, dass** das Auftragen des vorgenannten Extraktes aus Samen der Pflanze *Aframomum angustifolium* auf den Bereichen der Haut, die einer Anti-Aging-Pflege bedürfen, vorgenommen wird, insbesondere um die Folgen der Alterung, die aus der Wirkung der aktinischen Strahlungen, insbesondere der UV-Strahlen resultieren, zu bekämpfen.

15. Extrakt nach Anspruch 13, **dadurch gekennzeichnet, dass** die Anti-Aging-Aktivität insbesondere eine Anti-Falten-Aktivität, um das Auftreten von Falten zu verringern, zu beseitigen oder zu verlangsamen, eine Wirkung, welche die Struktur der Haut bewahrt oder wiederherstellt, insbesondere durch eine Wirkung zur Stimulation der Synthese von Kollagen, vor allem von Kollagen VII, das insbesondere im Bereich der dermo-epidermalen Junktionszone, oder DEJZ, vorhanden ist, und/oder durch eine reparierende Wirkung an den Oxytalan-Fasern und/oder den Elaunin-Fasern, eine Wirkung zum Schutz, zur Reparatur oder zur Wiederherstellung der Elastizität und/oder der Festigkeit der Haut betrifft.

## Claims

1. Cosmetic composition, **characterized in that** it comprises, as active ingredient, an extract of seeds of the *Aframomum angustifolium* plant obtained by an extraction method comprising at least one step of extraction of the ground seeds with an alcoholic or hydro-alcoholic solvent, in a cosmetically acceptable excipient.

2. Composition according to Claim 1, **characterized in that** the alcoholic or aqueous-alcoholic extract obtained is defatted by elimination of the lipids by means of at least one step consisting of extraction with an apolar organic solvent, for example hexane or n-heptane, thus obtaining a defatted alcoholic or aqueous-alcoholic extract.

3. Composition according to one of Claims 1 or 2, **characterized in that** the defatted alcoholic or aqueous-alcoholic extract constitutes in itself the active ingredient.

4. Composition according to either of Claims 2 or 3, **characterized in that** the defatted alcoholic or aqueous-alcoholic extract is subjected to a step consisting of decolouration by filtration over an appropriate decolouring agent, such as charcoal, and then, after elimination of the charcoal, washing with a solution of an alcoholic or aqueous-alcoholic solvent which may be the same as that which was used for the initial extraction, or different.

5. Composition according to one of Claims 1 to 4, **characterized in that** the extraction solvent is substantially completely evaporated off, obtaining a dry extract which can either be used as it is, as the abovementioned active ingredient, or dissolved once again in a cosmetically acceptable solvent, in particular an alcohol or an aqueous-alcoholic mixture, in order to obtain another variant of said active ingredient.

6. Composition according to one of Claims 1 to 5, **characterized in that** the alcohol or the alcohol used for the aqueous-alcoholic mixture is a mono-alcohol or a polyol containing from 1 to 4 carbon atoms.

7. Composition according to Claim 6, **characterized in that** said alcohol is chosen from methanol, ethanol, n-propanol, isopropanol, ethylene glycol, polypropylene glycol and butylene glycol.

8. Composition according to Claim 6 or 7, **characterized in that** said alcohol is ethanol.

9. Composition according to one of Claims 1 to 8, **characterized in that** the extract of *Longoza* seeds is present at a concentration, expressed on a dry extract basis, of between 0.001% and 5%, by weight relative to the total weight of the composition.

10. Composition according to one of Claims 1 to 9, **characterized in that** the extract of *Longoza* seeds is present at a concentration, expressed on a dry extract basis, of between 0.01% and 1%, by weight relative to the total weight of the composition.

11. Cosmetic agent in the form of an extract of seeds of the *Aframomum angustifolium* plant, said extract being as defined in any one of Claims 1 to 10.

12. Cosmetic agent according to Claim 11, **characterized in that** the extract of *Aframomum angustifolium* is used for an anti-ageing activity.

13. Extract of seeds of the *Aframomum angustifolium* plant, such as defined in any one of Claims 1 to 10 used in a process of cosmetic care by topical application on skin.

14. Extract according to claim 13, **characterized in that** it is performed the topical application of the extract of seeds of the *Aframomum angustifolium,* to the areas of the skin which are in need of an anti-ageing care, in particular for combating the effects of ageing resulting from the action of actinic radiation, in particular ultraviolet rays.

15. Extract according to Claim 13, **characterized in that** the anti-ageing activity is particularly aimed at an anti-wrinkle activity for decreasing, eliminating or slowing down the appearance of wrinkles, an action which preserves or restores the structure of the skin, especially by means of an action which stimulates the synthesis of collagen, in particular of collagen VII, in particular present at the dermal-epidermal junction, or DEJ, and/or by means of a repairing action on the oxytalan fibres and/or the elaunin fibres, an effect consisting of protection, repair or restoration of the elasticity and/or of the firmness of the skin.
